# EUROPÄISCHE PATENTANMELDUNG

(11) **EP 3 884 913 A1**
(43) Veröffentlichungstag der Anmeldung: **29.09.2021**
(21) Anmeldenummer: 20166211.1
(22) Anmeldetag: 27.03.2020
(51) Int. Cl.: A61F 5/41

(54) **PENISMANSCHETTE**

(71) Anmelder: Born, Oliver, 06862 Dessau-Rosslau (DE); Kothe, Andreas, 06886 Lutherstadt Wittenberg OT Griebo (DE); Klose, Thomas, 06869 Coswig (DE); Schädel, Heiko, 06366 Köthen (DE)
(72) Erfinder: Born, Oliver, 06862 Dessau-Rosslau (DE); Kothe, Andreas, 06886 Lutherstadt Wittenberg OT Griebo (DE); Klose, Thomas, 06869 Coswig (DE); Schädel, Heiko, 06366 Köthen (DE)
(74) Vertreter: Hertin und Partner Rechts- und Patentanwälte PartG mbB

(57) **Zusammenfassung**

Die Erfindung betrifft bevorzugt eine Penismanschette, welche aus einem hülsenförmigen Hohlkörper ausgestaltet ist. Der Hohlkörper weist zwei stirnseitige Öffnungen an den beiden Hohlkörperenden auf. Ferner ist der Betrag der Längsausdehnung des Hohlkörpers größer als sein Innendurchmesser. Der Hohlkörper ist im Querschnitt mindestens bereichsweise als ein C-Profil ausgestaltet, wodurch er entlang seiner Längsachse einen Schlitz aufweist. In einem weiteren Aspekt betrifft die Erfindung bevorzugt eine Verwendung der Penismanschette als Manschette für einen Penis beim Geschlechtsverkehr.

## Beschreibung

### Technisches Gebiet

Die Erfindung betrifft bevorzugt eine Penismanschette, welche aus einem hülsenförmigen Hohlkörper ausgestaltet ist. Der Hohlkörper weist zwei stirnseitige Öffnungen an den beiden Hohlkörperenden auf. Ferner ist der Betrag der Längsausdehnung des Hohlkörpers größer als sein Innendurchmesser. Der Hohlkörper ist im Querschnitt mindestens bereichsweise als ein C-Profil ausgestaltet, wodurch er entlang seiner Längsachse einen Schlitz aufweist. Auf der gegenüberliegenden Seite des Schlitzes weist der Hohlkörper eine Wanddicke von mindestens 8 mm auf. In einem weiteren Aspekt betrifft die Erfindung bevorzugt eine Verwendung der Penismanschette als Manschette für einen Penis beim Geschlechtsverkehr.

### Hintergrund und Stand der Technik

Im Stand der Technik sind verschiedene Hohlkörper beschrieben, die über die primären männlichen äußeren Geschlechtsmerkmale gestülpt bzw. auf- oder um diese positioniert werden können.

Seit langem sind Penishüllen oder Penisringe bekannt, welche für eine erhöhte Stimulanz beim Geschlechtsverkehr sorgen.

Es ist kein unbeschriebenes Problem, dass bedingt durch einen zu kleinen Durchmesser des Penis eines Mannes die Sexualpartnerin / der Sexualpartner nicht befriedigt werden kann. Studien belegen auch, dass diese Situation psychisch belastend für die betreffenden Männer sein kann und diese Männer einem hohen Leidensdruck ausgesetzt sind. Ferner können Spannungen in Partnerschaftsbeziehungen ausgehend von einem nicht erfüllenden bzw. befriedigendem Sexualleben hervortreten.

Penishüllen oder Penisringe können demnach über den Penis gezogen werden, wodurch sie für eine künstliche Verdickung und/oder Verlängerung des Penis sorgen.

Im weiteren Dokument werden Sexualpartner geschlechtlich nicht getrennt aufgeführt. Es fallen männliche, weibliche und diverse Sexualpartner/innen unter den Begriff Sexualpartner.

In der DE 20 2018 105 051 U1 wird eine solche Penishülle offenbart. Derartige Penishüllen haben jedoch den Nachteil, dass sie aufgrund ihrer geschlossenen Ring- oder Hüllenform den intimen Kontakt zwischen den Sexualpartnern stark vermindern. Die freilegende Penisfläche begrenzt sich dabei nur auf die Peniseichel. Insbesondere beim Träger einer solchen Penishülle kommt es zu einem vermindert natürlichen Gefühl, wodurch sich das sexuell befriedigende Erlebnis für den Penishüllenträger stark reduziert.

Überdies eignen sich vollumschlossene Penishüllen oder Penisringe aus dem Stand der Technik nur begrenzt für unterschiedliche Penisdicken sowohl im nicht erigierten als im erigierten Zustand des Penis, da ihr Formänderungsvermögen mit zunehmender Dehnung abnimmt und somit die natürliche Penisverdickung durch eine Erektion behindert, so dass der äußere Druck auf den Penis mit zunehmender Penisdicke in unerwünschter Weise zunimmt. Die Verwendung einer zu kleinen Penishülle oder zu kleinem Penisring kann im schlimmsten Fall zu ernsthaften Verletzungen führen. Wenn sich die Hülle oder der Ring nicht mehr vom Penis lösen lässt, hat das im Penis aufgestaute Blut keine Möglichkeit mehr abzufließen, sodass unter Umständen ein Arzt aufgesucht werden muss, der den Ring oder die Hülle durchtrennt. Andernfalls besteht die Gefahr einer Amputation des Penis.

Des Weiteren müssen die genannten Penishüllen in der Regel über die Peniseichel und den Penisschaft gezogen werden, was der Stimulanz des Penis beim Geschlechtsverkehr abträglich ist, da der fühlbare Kontakt beim Geschlechtsverkehr an den Stimulanzregionen des Penis - insbesondere der Eichel - durch zu große Fremdmaterialanhäufungen in diesen Regionen gestört bzw. verhindert wird.

Darüber hinaus ist es bekannt Penishüllen im medizinischen Bereich beispielsweise Inkontinenz einzusetzen oder als Nottoilette bzw. als äußerer Harnkatheter zu verwenden. Die DE 37 41 557 C2 beschreibt einen äußeren Harnkatheter, der eine Penishülle aufweist. Diese werden beispielsweise bei männlichen Patienten mit Rückenmarksverletzungen eingesetzt, da diese inkontinent sein können und nicht immer sogenannte innere Katheter vertragen. Auch die EP 05 28 965 D1 betrifft eine Penishülle als Inkontinenz-Vorrichtung. In der DE 100 56 858 D4 wird eine mobile Not- oder Ersatztoilette zu den Urnieren beschrieben, die eine Penishülle aufweist.

Zur Behandlung von erektiler Dysfunktion genutzt sind Penispumpen bekannt. Eine Penispumpe ist eine Penishülle ausgestattet mit einer Unterdruckpumpe, welche zusätzliches Blut in den Penis befördert, wodurch dieser sich versteift und mehr Volumen annimmt. Um die Erektion wirksam während des Geschlechtsverkehrs zu halten, sind Stauringe notwendig, welche beim Geschlechtsverkehr hinderlich sein können und die Erlebnisqualität mindern.

Weitere medizinische Unterstützungen zur Behandlung von erektiler Dysfunktion, welche im Vergleich zu bekannten Penispumpen einen simplen Aufbau aufweisen, sind ebenfalls bekannt.

Die DE 3723746 A1 offenbart eine Impotenzstütze, welche als eine mit einem Schlitz versehende Manschette ausgestaltet ist. Die Öffnung eines ersten Endes in Peniseichelnähe ist dabei im Durchmesser kleiner als die Öffnung der restlichen Manschette ausgestaltet. Der Manschettenkörper ist aus Kunststoff und umfasst eine homogene und konstante Wanddicke, wobei alle Kanten mit lippenförmigen Abrundungen ausgestaltet sind. Diese Abrundungen sind aus Klebstoffen oder Füllungsstoffen. Die beschriebene Impotenzstütze weist eine überaus geringe Wanddicke auf, damit sie unauffällig dem Träger eine medizinische Unterstützung beim Geschlechtsverkehr darbietet. Die gesamte Manschette ragt dabei radial nicht über den Umfang der Peniseichel hinaus, sondern sie liegt hinter der Eichel versteckt am Schaft vor. Durch die geringe Wanddicke ist die Manschette nur zum medizinischen Zwecke anwendbar. Eine erhöhte Stimulanz bei einem Sexualpartner neben der medizinischen Unterstützung beim Manschettenträger kann dabei nachteilig nicht erreicht werden, weil die Manschette zu keiner wesentlichen künstlichen Vergrößerung des Penisdurchmessers führt.

Die Patentschrift US 4 615 337 A offenbart ebenfalls eine Manschette, die als Unterstützung bei Erektionsschwäche dienen soll. Die Manschette umfasst einen Schlitz, sodass die Manschette im Querschnitt als C-Profil ausgestaltet ist, wobei die Schenkel des C'S an den Manschettenenden länger als im Mittelbereich ausgestaltet sind. Die Manschette wird insbesondere durch die verlängerten C-Schenkel am Penis gehalten. Das Material der Manschette ist bevorzugt ein formbarer Kunststoff (Polyethylen) mit einer Dicke von ca. 1 mm. Auch diese Manschette ermöglicht durch die geringe Manschettendicke nachteilig keine wesentliche künstliche Penisverdickung, sodass eine vermehrte Stimulation des Sexualpartners nicht möglich ist.

Die DE 72 43 079 U offenbart eine Penismanschette, die mehrere Schichten Latex oder Kautschuk umfasst. In den Lagen sind Plastikplättchen eingearbeitet, die der Manschette eine ausreichende Steifheit geben sollen. Entlang seiner Längsrichtung erstreckt sich ein Spalt variabler Größe. Hilfsmittel bzw. Befestigungsmechanismen (verschiedene Bänder) können zu einer Unterstützung der Halterung dienen. Wie die beiden vorhergehenden beschriebenen Dokumente beschreibt diese Offenbarung nur ein Instrument zur Unterstützung bei einer Erektionsschwäche. Die Wandstärke ist so nachteilig gering wie möglich gehalten und zielt nicht auf die Stimulanz eines Sexualpartners aufgrund einer künstlichen Penisverdickung ab.

### Aufgabe der Erfindung

Aufgabe der Erfindung ist es somit die Nachteile des Standes der Technik zu beseitigen und eine Penishülle bereitzustellen, die dem Träger während des Geschlechtsverkehrs ein besonders natürliches Gefühl verleiht und eine künstliche für den Geschlechtspartner stimulierende Penisverdickung bewirkt, wobei ein störender äußerer Druck mit zunehmender Penisdicke verhindert und, insbesondere beim Anbringen der Penishülle, Fremdmaterialanhäufungen an der Peniseichel vermieden werden soll.

### Zusammenfassung der Erfindung

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

In einem ersten Aspekt betrifft die Erfindung bevorzugt eine Penismanschette, aufweisend einen hülsenförmigen Hohlkörper mit jeweils einer stirnseitigen Öffnung an beiden Hohlkörperenden, wobei die Länge des Hohlkörpers größer ist als sein Innendurchmesser, und der Hohlkörper im Querschnitt mindestens bereichsweise als ein C-Profil ausgestaltet ist, wodurch der Hohlkörper entlang seiner Längsachse einen Schlitz aufweist dadurch gekennzeichnet, dass der Hohlkörper auf der gegenüberliegenden Seite des Schlitzes eine Wanddicke von mindestens 8 mm aufweist.

Eine solche Penismanschette eignet sich besonders gut für den Geschlechtsverkehr. Die Manschette kann spontan und in überaus schneller Weise um einen Penisschaft positioniert werden. Es war für die Erfinder überraschend und nicht vorherzusehen, dass die Penismanschette auch bei besonders bewegungsintensivem Geschlechtsverkehr positionsfest ohne anderweitige Befestigungsmittel am Penis verbleibt. Besonders vorteilhaft vergrößert die angelegte Penismanschette den Durchmesser des Penis und führt demnach zu einer erhöhten Stimulanz des Sexualpartners. Dies wirkt sich insbesondere vorteilhaft auf die Lebensqualität von Männern (und deren Sexualpartnern) aus, die bedingt durch einen zu kleinen Durchmesser des Penis den Sexualpartner nicht befriedigen können. Durch den im Vergleich zum Stand der Technik beschriebenen Penishülsen weist die bevorzugte Penismanschette ein erhöhtes Gefühl an Natürlichkeit beim Träger der Penismanschette auf, da der Penis durch den offenen Schlitz der Manschette zu einem großen Teil frei liegt und dadurch zu einem großen Teil direkter Kontakt zwischen dem Penis und dem Sexualpartner ermöglicht wird. Im Gegensatz zu den im Stand der Technik beschriebenen Schlitz aufweisenden Erektionshilfen ist die erfindungsgemäße Penismanschette durch die erhöhte Wanddicke besonders vorteilhaft für die Stimulanz eines Sexualpartners geeignet. Ferner zeigt die Manschette aufgrund der bevorzugten Wanddicke auf der gegenüberliegenden Seite des Schlitzes eine besonders hohe Stabilität auf, sodass experimentierfreudiger Sexualverkehr ermöglicht wird, ohne dass die Manschette Schäden davonträgt.

Ein weiterer Vorteil besteht darin, dass das Blut im Penis aufgestaut wird, wodurch längere sexuelle Aktivitäten möglich sind. Dabei übt die Penismanschette überraschenderweise - im Gegensatz zu beispielsweise bekannten Stauringen - keinen unangenehmen Druck auf den Träger aus und ist zudem durch den aufweisenden Schlitz vorteilhaft zu jederzeit sehr gut vom Penis lösbar. Im Bereich der Körperöffnungen, die mit der Penishülle interagieren, kommt es zu einer überraschend verbesserten Blutzirkulation. Insbesondere experimenteller Sex benötigt einen größeren Zeitraum, damit beide Partner ihn erlernen und mit Gewinn umsetzen können.

Ferner, besteht der Vorteil der Erfindung darin, dass die Vorrichtung sowohl zur Volumensteigerung oder Vergrößerung um den Penis genutzt werden kann, sodass auch Paare mit deutlich verschieden groß dimensionierten primären und sekundären Geschlechtsmerkmalen in einer stressreduzierten Art und Weise sexuell aktiv sein können.

Als ein zusätzlicher Vorteil kann die Penismanschette, der Eingangs genannten Art sich auch unterstützen bei der Behandlung von erektiler Dysfunktion eignen. Für die Behandlung einer derartigen Erkrankung werden unter anderem hydraulische Schwellkörperimplantate oder biegsame Penisprothesen verwendet.

Ein hydraulisches Schwellkörperimplantat ist eine Form einer Prothese, welche aus den eigentlichen Implantaten, einer Pumpe, einem Ballon mit steriler Kochsalzlösung und diversen Schlauchleitungen ausgestaltet ist. Die Pumpe, welche im Hodensack eingepflanzt wird, ist mit dem Wasserballon (im Bauchraum untergebracht) und den künstlichen Schwellkörpern im Penis verbunden. Mittels der Pumpe wird die sterile Kochsalzlösung in die künstlichen Schwellkörper gepumpt, dadurch kommt es zur Erektion des Penis. Durch ein Ventil am Hodensack kann das Wasser wieder in den Ballon zurückgeführt und die Erektion damit beendet werden.

Bei biegsamen Penisprothesen handelt es sich um künstliche Schwellkörper aus Silikon, die anstelle der natürlichen Schwellkörper eingesetzt werden und von Hand in die gewünschte Position verbogen werden können.

Die Stabilität von derartigen Prothesen ist, insbesondere bei bewegungsintensivem und experimentellem Geschlechtsverkehr, nicht ausreichend. So wirkt die bevorzugte Penismanschette bei Ihrer Verwendung nicht nur aufgrund der Verdickung stimulierend, sondern zudem vorteilhaft unterstützend und ermöglicht der Prothese eine erhöhte Stabilität.

Der erfindungsgemäße hülsenförmige Hohlkörper weist eine Innenfläche und eine Außenfläche auf. Dem Fachmann ist bekannt, dass unter einem hülsenförmigen Hohlkörper auch ein schlauch- oder rohrförmiger Körper zu verstehen ist.

In einer bevorzugten Ausführungsform ist die Penismanschette so ausgestaltet, dass die Innenfläche des Hohlkörpers an die Größe eines erigierten Penis angepasst ist. Die Außenfläche des Hohlkörpers hingegen umfasst eine freie Ausgestaltung. Eine freie Ausgestaltung kann je nach Vorliebe der Ausgestaltung zu einer erhöhten Stimulanz beim Sexualpartner führen. Insbesondere eine nicht gleichmäßig ausgestaltete Außenfläche hat überraschend zu besonders erhöhter Erregung des Sexualpartners geführt.

In einer bevorzugten Ausführungsform ragt die Außenfläche der Penismanschette radial mindestens bereichsweise über den größtmöglichen Umfang der Peniseichel hinaus und erweist sich daher vorteilhaft als besonders umfangreiche künstliche Penisverdickung.

Erfindungsgemäß sind die stirnseitigen Öffnungen bevorzugt kreisförmig ausgestaltet. Die Öffnungen weisen bevorzugt insbesondere einen Durchmesser auf, der dem Betrage nach etwa dem Innendurchmesser des hülsenförmigen Körpers gleicht, wobei der Mittelpunkt der kreisförmigen Öffnung auf der Längsachse des Hohlkörpers liegt.

In einer weiteren bevorzugten Ausführungsform sind die stirnseitigen Öffnungen oval oder eckig ausgestaltet. Dies führt vorteilhaft zu einer besseren Befestigung der Penismanschette an den Penisschaft, da der Penisschaft eines Mannes lokale Äderungen aufweisen kann.

Begriffe wie im Wesentlichen, ungefähr, etwa, ca. etc. beschreiben bevorzugt einen Toleranzbereich von weniger als ± 40%, bevorzugt weniger als ± 20%, besonders bevorzugt weniger als ± 10 %, noch stärker bevorzugt weniger als ± 5% und insbesondere weniger als ± 1% und umfassen stets den exakten Wert. Ähnlich beschreibt bevorzugt Größen die ungefähr gleich sind. Teilweise beschreibt bevorzugt zu mindestens 5 %, besonders bevorzugt zu mindestens 10 %, und insbesondere zu mindestens 20 %, in einigen Fällen zu mindestens 40 %.

Dem Fachmann ist die Ausgestaltung eines C-Profils im Zusammenhang mit Hohlkörpern bekannt. Das C-Profil ist dabei bevorzugt über die gesamte Länge der Penismanschette ausgestaltet. Ein C-Profil ist bevorzugt so definiert, dass ein Mittelteil durch zwei Schenkel erweitert ist, wobei beide Schenkel beabstandet und eine spiegelverkehrte Ausgestaltung aufweisen.

In einer bevorzugten Alternative ist nur ein Teilbereich entlang der Längsachse der Penismanschette als C-Profil ausgestaltet, wobei der restliche Teil beispielsweise auch durchgehend geschlossen sein kann und ein O-Profil ausmacht. Ein O-Profil ist dem Fachmann aus dem Stand der Technik bekannt.

Bevorzugt beträgt ein Teilbereich als C-Profil entlang einer Längsachse jedoch mindestens 2/10, 3/10, 4/10 5/10, 6/10, 7/10, 8/10 oder mehr, der gesamten Länge der Penismanschette. Die Mischform aus einem Teilbereich ausgestaltet durch ein C-Profil und ein weiterer Teil ausgestaltet aus einem O-Profil zeigt besonders vorteilhafte positionsfestsitzende und stabile Eigenschaften, so dass auch besonders experimentierfreudige Sexpraktiken vollzogen werden können. Hierbei ist die Ausgestaltung der Teilbereiche in jeder Konstellation und Kombination möglich. So können beispielsweise auch zwei beabstandete Teilbereiche als O-Profil und ein dazwischen liegender Teilbereich durch ein C-Profil ausgestaltet sein oder umgekehrt.

In eine weiteren bevorzugten Ausführungsform der Penismanschette weist der Schlitz bevorzugt eine Schlitzbreite von 5mm bis 80mm auf, stärker bevorzugt eine Schlitzbreite von 15mm bis 50mm und besonders stark bevorzugt 25mm bis 40mm.

Der Schlitz ist durch den Abstand der beiden Schenkel des C-Profils im Querschnitt definiert und abhängig vom Gebrauchs- bzw. Verwendungszustand oder Nichtgebrauchszustand der Penismanschette. Im Verwendungszustand der Penismanschette nimmt die Schlitzbreite maximal die Größe des Penisdurchmessers, um den die Penismanschette angebracht vorliegt, an. Demnach kann der Schlitz in diesen Fällen eine Schlitzbreite von 5mm bis 80mm aufweisen. Bei diesen Schlitzbreiten sind insbesondere auch Fälle von besonders großen und besonders kleinen Penisdurchmessern erfasst.

In einer weiteren bevorzugten Ausführungsform ist die Penismanschette dadurch gekennzeichnet, dass der Hohlkörper Silikon umfasst. Silikon ist vorteilhaft ungiftig sowie geruchslos. Zudem ist Silikon vorteilhaft geeignet für Allergiker, UV-beständig, wasserfest und wasserdicht. Unter Silikon ist auch das für den Fachmann bekannte medizinische Silikon umfasst.

In einer weiteren bevorzugten Ausführungsform umfasst der Hohlkörper Latex, insbesondere Naturlatex, welches vorteilhafterweise keine Weichmacher und/oder wenig Allergene aufweist. Latex erweist sich vorteilhaft gut verträglich und weist angenehmes Körpergefühl auf

In einer weiteren bevorzugten Ausführungsform umfasst der Hohlkörper ein biokompatibles Material, ausgewählt aus der Gruppe, die aus Celluloseether, Cellulose, Celluloseester, fluoriertem Polyethylen, Naturkautschuk, phenolischem Material, Poly-4-methylpenten, Polyacrylonitril, Polyamid, Polyamidimid, Polyacrylat, Polybenzoxazol, Polycarbonat, Polycyanoarylether, Polyester, Polyestercarbonat, Polyether, Polyetheretherketon, Polyetherimid, Polyetherketon, Polyethersulfon, Polyethylen, Polyfluorolefin, Polyimid, Polyolefin, Polyoxadiazol, Polyphenylenoxid, Polyphenylensulfid, Polypropylen, Polystyrol, Polysulfid, Polysulfon, Polytetrafluorethylen, Polythioether, Polytriazol, Polyurethan, Polyvinyl, Polyvinylidenfluorid, regenerierter Cellulose, Silikon, Urea-Formaldehyd oder Copolymeren oder physikalischen Gemischen hiervon.

In einer weiteren bevorzugten Ausführungsform der Erfindung umfasst der Hohlkörper ein Material mit einem Elastizitätsmodul (bei 20°C Umgebungstemperatur) von bevorzugt 0,1 MPa bis 7*10³ MPa, stärker bevorzugt 0,3 bis 3*10³ MPa und besonders stark bevorzugt 30 MPa bis 1*10³ MPa.

In einer weiteren Ausführungsform umfasst der Hohlkörper ein Material mit einer Shore-Härte bevorzugt von 0 bis 70 Shore-A, stärker bevorzugt 5 bis 40 Shore-A und besonders stark bevorzugt von 10 bis 30 Shore-A. Dem Fachmann ist bekannt, dass die Shore-Härte ein Werkstoffkennwert für Elastomere und Kunststoffe ist und in den Normen DIN EN ISO 868, DIN ISO 7619-1 und ASTM D2240-00 festgelegt ist.

In einer weiteren bevorzugten Ausführungsform ist die Penismanschette dadurch gekennzeichnet, dass der Hohlkörper ein eingebettetes Stützskelett umfasst. Das Stützskelett verleiht der Penismanschette vorteilhaft Halt und Stabilität. Überdies verleiht das Stützskelett der Penismanschette vorteilhafterweise seine elastischen Eigenschaften.

Erfindungsgemäß handelt es sich bevorzugt um ein Endoskelett, welches in den Hohlkörper eingebettet ist. Ein Endoskelett ist vorteilhaft innerhalb der Penismanschette angeordnet, sodass das Hohlkörpermaterial - bevorzugt Silikon - das Stützskelett ummantelt.

In einer weiteren bevorzugten Ausführungsform ist die Penismanschette dadurch gekennzeichnet, dass das Stützskelett eine aus einem einzigen Bauteil ausgestaltete Klammer ist, wobei die Klammer bereichsweise ebenfalls einen als C-Profil ausgestalteten Querschnitt aufweist wodurch die Klammer entlang ihrer Längsachse auch einen Klarmmerschlitz aufweist. Eine aus nur einem Bauteil hergestellte Klammer ist vorteilhaft günstig in der Herstellung und überdies für besonders starke Belastungen geeignet, da sie keine Materialschwachstellen insbesondere in Form von Verbindungselementen aufweist. Demnach ist eine Penismanschette ausgestaltet mit einer derartigen Klammer vorteilhaft für besonders experimentierfreudige sexuelle Aktivitäten.

In einer Ausgestaltung umfasst ein Flächenträgheitsmoment, bevorzugt ein axiales Flächenträgheitsmoment, des C-Profils der Klammer bevorzugt 3 000 mm⁴ bis 20 000 000 mm⁴; stärker bevorzugt 50 000 mm⁴ bis 50 000 000 mm⁴ und besonders stark bevorzugt 300 000 mm⁴ bis 1 000 000 mm⁴.

Dadurch das die Klammer bevorzugt ebenfalls im Querschnitt als C-Profil ausgestaltet ist, wird dem Hohlkörper eine besonders starke Stabilität zugeführt, da das Skelett über den gesamten Querschnitt, des Hohlkörpers ausgestaltet ist. Durch die Bauweise der Klammer - im Querschnitt als ein C-Profil - wird eine Klammerkraft ermöglich, indem die Schenkel des C's jeweils eine entgegengesetzt nach außen gerichteter Zugkraft oder jeweils eine entgegengesetzte nach innen gerichtete Druckkraft erfahren. Die Klammer wirkt wie eine Feder und zielt darauf ab, durch ihr elastisches Verhalten, in die Ausgangsposition zurück zu gelangen.

In einer weiteren bevorzugten Ausgestaltung umfasst die Klammer eine Vielzahl von Bauteilen, wobei die Bauteile über Verbindungsmittel miteinander verbunden sind. Die verschiedenen Bauteile sind bevorzugt aus einem gleichen Material ausgestaltet, stärker bevorzugt können sie aus unterschiedlichen Materialien ausgestaltet sein In einer weiteren bevorzugten Ausführungsform ist die Klammer so ausgestaltet, dass sie ausgehend des Bereichs des C-Profils zwei in axialer Richtung ausgebildete Fortsätze aufweist. Bevorzugt weist die Klammer 1 bis 3 Fortsätze auf, insbesondere 2. Durch die Ausgestaltung von Fortsätzen wird vorteilhaft Material bei der Produktion der Klammer gespart, da die Klammer nicht durchgehend als C-Profil ausgestaltet ist. Für die Erfinder war es überraschend, dass das benötigte elastische Verhalten der Klammer dabei beibehalten wurde. Je nach Ausgestaltung der Fortsätze, kann vorteilhaft eine Stabilität sowie elastisches Verhalten der Penismanschette den geforderten Anforderungen angepasst werden.

In einer weiteren bevorzugten Ausführungsform der Erfindung ist die Penismanschette dadurch gekennzeichnet, dass die Klammer vollständig mit einer Schichtdicke von 1 mm bis 50mm, stärker bevorzugt 2 mm bis 25 mm Silikon ummantelt ist, wobei die Klammer auf ihrer nach außen gewandten Seitenfläche (16) der gegenüberliegenden Seite des Schlitzes (13) eine Schichtdicke von mindestens 5mm aufweist auf. Es hat sich gezeigt, dass sich durch die vorgenannten Schichtdicken ein besonders weiches und angenehmes Gefühl beim Träger der Penismanschette einstellt. Insbesondere verspürt ein Träger durch den benannten Schichtdickenbereich nicht das im Hohlkörper eingebettete Skelett aus wesentlich härterem Material als das ummantelte Silikon.

In einer bevorzugten Ausführungsform ist die Schichtdicke, welche sich auf der Innenseite der Klammer befindet konstant. Die Schichtdicke auf der nach außen gewandten Seitenfläche der Klammer ist hingegen bevorzugt nicht konstant.

In einer weiteren bevorzugten Ausführungsform ist die Penismanschette dadurch gekennzeichnet, dass die Schichtdicke nicht konstant ist. Die Schichtdicke kann beispielsweise über die Längsachse des Hohlkörpers inhomogen bzw. nicht konstant sein. Eine nicht konstant ausgebildete Schichtdicke über die gesamte Länge ermöglicht eine besonders natürliche Formen der Außenfläche.

In einer weiteren bevorzugten Ausführungsform verändert sich die Schichtdicke radial im Querschnitt auf der nach außen gewandten Seitenfläche der Klammer, sodass besonders bevorzugt die Schichtdicke den größten Wert auf der nach außen gewandten Seitenfläche der Klammer im Bereich der gegenüberliegenden Seite des Schlitzes aufweist. Eine derartige sichelförmige Ausgestaltung erweist sich vorteilhaft als enorm stabiltätssteigernd und ermöglicht eine erhöhte Lebensdauer. Zudem wirkt die vergrößerte Materialanhäufung auf der gegenüberliegenden Seite des Schlitzes stimulierend für einen Sexualpartner beim Geschlechtsverkehr, da die Manschette vorzugsweise radial über den größtmöglichen Umfang der Peniseichel hinausragt. Neben der Stimulation des Sexualpartners hat sich vorteilhaft gezeigt, dass ein vergrößerter einseitiger Druck über die Seite mit der größten Wanddicke auf den Penis, während des Ein- und Ausführens des Penis in eine Körperöffnung, übertragen wird, welcher sich vorteilhaft stimulierenden für den Manschettenträger auswirken kann.

In einer weiteren bevorzugten Ausführungsform weist die Schichtdicke auf der nach außen gewandten Seitenfläche der Klammer im Bereich der gegenüberliegenden Seite des Schlitzes einen Wert von bevorzugt mindestens 7mm, stärker bevorzugt mindestens 12mm, besonders stark bevorzugt mindestens 15mm, vielmehr bevorzugt mindestens 17mm und insbesondere mindestens 27mm.

In einer weiteren bevorzugten Ausführungsform weist die auf der nach außen gewandten Seitenfläche der Klammer im Bereich der gegenüberliegenden Seite des Schlitzes einen Wert von bevorzugt maximal 67 mm, stärker bevorzugt maximal 63mm, besonders stark bevorzugt maximal 55mm, vielmehr bevorzugt maximal 45mm und insbesondere maximal 37mm.

In einer weiteren bevorzugten Ausführungsform verändert sich die Schichtdicke des Hohlkörpers entlang der seiner Längsachse. In beiden Endbereichen des Hohlkörpers sind bevorzugt kleine Schichtdicken vorgesehen, wobei diese sich beide nach außen entlang der Längsachse verjüngen. Die gegenüberliegende Seite des Schlitzes weist dabei weiterhin die größtmögliche Schichtdicke auf.

In einer weiteren bevorzugten Ausführungsform ist der Innendurchmesser der Klammer im Bereich des C-Profils größer als der Innendurchmesser des Hohlkörpers. Dadurch ist vorteilhaft der Innenbereich vollständig aus einem weichen Material ausgestaltet, was dem Träger einer Penismanschette zu einem angenehmen Gefühl verhilft.

In weiteren bevorzugten Ausführungsformen kann das Material, welches die Klammer ummantelt, aus einem andersartigen Material, anstatt von Silikon, ausgestaltet sein. Materialien und Kennwerte, die den Hohlkörper umfassen können, sind weiter oben im Dokument beschrieben.

In einer weiteren bevorzugten Ausführungsform ist die Penismanschette dadurch gekennzeichnet, dass die Klammer eine Vielzahl von Öffnungen aufweist. Durch die hohe Anzahl an Öffnungen, wird eine stärkere Verbindung der Klammer mit dem übrigen Material, in welchem die Klammer eingebettet ist, hergestellt, sodass die Penismanschette sehr hohen Belastungen ausgesetzt werden kann und trotzdem sehr langlebig ist. Zusätzlich wirken sich die Öffnungen materialsparend in Bezug auf die Herstellung der Klammern aus.

Der Fachmann versteht unter einer eingebetteten Klammer unter anderem auch eine eingegossene Klammer. Die eingebettete bzw. eingegossene Klammer weist durch die Öffnungen vorteilhaft bessere Belastungseigenschaften in Bezug auf aufkommende Axialkräfte auf, da die Klammer eine stärkere Verbindung zu seinem umgebenden Material eingeht.

In einer bevorzugten Ausgestaltung weisen die Öffnungen bevorzugt eine geometrische Form auf ausgesucht aus der Gruppe (ohne darauf beschränkt zu sein): Ellipse, Dreieck, Viereck (bspw. Quadrat, Rechteck, Parallelogramm, Raute), Fünfeck, Sechseck, jegliche Polygone etc. Besonders bevorzugt sind die Öffnung als Kreis ausgestaltet. Durch eine einfache Bohrung lässt sich diese vorteilhaft ohne großen Aufwand in einem separaten Fertigungsschritt umsetzen. Alternativ lassen sich derartige Öffnungen in einem Urformverfahren wie bspw. dem Spritzgießen in einer Gussform oder dem 3D-Druckverfahren ausgestalten. Dem Fachmann sind diese Verfahren bekannt.

In einer weiteren bevorzugten Ausgestaltung ist die Formgebung der einzelnen Öffnungen im Wesentlichen kongruent. Die Öffnungen können jedoch auch eine unterschiedliche Formgebung aufweisen.

In einer bevorzugten Ausführungsform ist die Penismanschette dadurch gekennzeichnet, dass die Öffnungen gleichmäßig über die gesamte Klammer verteilt sind. Durch gleichmäßig angeordnete Öffnungen über die gesamte Klammer erhöht sich die Lebensdauer der Penismanschette, da die Verbindung zwischen Klammer und umgebendes Material gleichmäßig besonders stark ausgeprägt ist und daher kein Bereich mit einer Schwachstelle ausgeprägt ist.

In einer weiteren Ausführungsform der Erfindung ist die Penismanschette dadurch gekennzeichnet, dass die Klammer aus einem Material gefertigt ist, welches Stützmaterial, bevorzugt Holz, Metall oder Kunststoffe, umfasst. Ein solches Stützmaterial dient besonders vorteilhaft zur Festigkeit und Steifigkeit der Penismanschette.

Erfindungsgemäß ist ein Stützmaterial bevorzugt ein Material mit einem Elastizitätsmodul (bei 20°C Umgebungstemperatur) von bevorzugt 1*10³ MPa bis 100*10³ MPa, stärker bevorzugt 3,5*10³ MPa bis 70*10³ MPa. Insbesondere diese Materialkennwerte dienen zur vorteilhaften Biegesteifigkeit der Klammer sowie der Penismanschette.

In einer weiteren bevorzugten Ausführungsform weist das Stützmaterial eine Härte von mindestens 50 Shore A, bevorzugt mindestens 70 Shore A auf. Durch die bevorzugte Härte kann eine besonders gute Stabilisierung erreicht werden, ohne dass die beim Geschlechtsverkehr durchaus gewünschte Flexibilität der Penismanschette nachteilig leidet. Vorteilhaft kann für Kunststoffe derartige Härte auf einfache Weise eingestellt bzw. gewährleistet. Ebenso weisen vorteilhaft auch Holz, Metall sowie härtere Verbundstoffe Härten auf, welche die oben genannten Grenzparameter überschreiten. In einer weiteren bevorzugten Ausführungsform weist die Klammer eine Biegesteifigkeit von bevorzugt 5*10⁶ N mm² bis 2*10¹² N mm², stärker bevorzugt von 14*10⁸ N mm² bis 7*10¹⁰ N mm². Überraschenderweise haben sich insbesondere die beschriebenen Werte vorteilhaft für eine besonders gute Stabilität der Klammer erwiesen. Die Klammer weist bei diesen Werten zudem ein besonders hervorragendes elastisches Verhalten auf.

In einer weiteren Ausführungsform umfasst die Klammer bevorzugt Holz oder Metall, stärker bevorzugt Kunststoff. In einer weiteren Variante umfasst die Klammer jegliche aufgeführten Materialien mit Faserverstärkungen (sog. Faserverbundstoffe). Auch der restliche Hohlkörper kann Faserverstärkungen umfassen.

In einer bevorzugten Ausgestaltung ist der Kunststoff bevorzugt ausgesucht aus der Gruppe (ohne darauf beschränkt zu sein): Polyoxymethylen, Polyethylen, Polypropylen, Polyamid, Polyethylenterephthalat, Polybutylenterephthalat, Acrylnitril-Butadien-Styrol, Polymethylmethacrylat, Polystyrol, Polyvinylchlorid, Polycarbonat, Styrol-Acrylnitril-Copolymer, Polyphenylenether, Naturkautschuk, Acrylnitril-Butadien-Kautschuk, StyrolButadien-Kautschuk, Chloropren-Kautschuk, Butadien-Kautschuk und Ethylen-Propylen-Dien-Kautschuk.

In einer weiteren Ausführungsform umfasst die Klammer ein Kunststoff mit einer Shore-Härte bevorzugt von 50 bis 100 Shore-A. Demnach verleiht eine hohe Härte des Stützskeletts bzw. der Klammer dem Hohlkörper eine vorteilhafte Stabilität, Steifigkeit und Festigkeit.

In einer weiteren Ausführungsform der Penismanschette ist die Klammer aus einem härteren Material ausgestaltet als ihr umgebendes Material im Hohlkörper.

In einer weiteren Ausführungsform der Penismanschette ist die Klammer aus einem Material mit einem höheren Elastizitätsmodul ausgestaltet als ihr umgebendes Material im Hohlkörper.

In einer weiteren bevorzugten Ausgestaltung ist die Penismanschette dadurch gekennzeichnet, dass die Längsausdehnung des Hohlkörpers im Wesentlichen der Länge eines Penisschafts gleicht. Die Peniseichel gehört zu den stärksten erogenen Zonen eines Penis. Durch die bevorzugte Ausgestaltung bleibt diese frei von Fremdmaterial, sodass ein natürliches stimulierendes Gefühl beim Träger einer Manschette beibehalten wird.

Im Sinne der Erfindung ist der Penisschaft der Teil des Penis, der äußerlich sichtbar vom Becken eines Mannes bis zur der Peniseichel ausgeprägt ist.

Die Längsausdehnung der Penismanschette weist demnach eine Längsausdehnung von bevorzugt 30mm bis 250mm, stärker bevorzugt 50mm bis 180mm und besonders stark bevorzugt von 80mm bis 150mm auf.

In einer weiteren bevorzugten Ausführungsform ist die Penismanschette dadurch gekennzeichnet, dass der Innendurchmesser des Hohlkörpers eine Größe von 20 mm bis 40 mm, bevorzugt 25 mm bis 33mm aufweist. Diese Innendurchmesser sind insbesondere vorteilhaft, um einen Penisschaft durch die Penismanschette zu klammern, wobei kein unangenehmer Druck auf den Penis ausgeübt wird.

Im Sinne der Erfindung ist der Innendurchmesser des Hohlkörpers reversibel veränderbar, insbesondere vergrößerbar. Im Nichtgebrauch der Penismanschette nimmt der Innendurchmesser des Hohlkörpers eine Größe von bevorzugt 20 mm bis 40 mm, bevorzugt 25 mm bis 33mm an. Bei der Positionierung um einen Penisschaft wird der Innendurchmesser bevorzugt erweitert und nimmt die Größe des Penisdurchmessers an, wobei durch das elastische Verhalten der Penismanschette (aufgrund der eingebetteten Klammer) eine rückwirkende Klammerkraft ausgebildet wird. Diese Klammerkraft ist für die feste Positionierung der Penismanschette an den Penisschafft verantwortlich. Auch ein Abrutschen des Penis nach Abklingen der Erektion wird durch die erfindungsgemäße Vorrichtung verhindert.

Statistiken haben ergeben, dass der durchschnittliche Penisdurchmesser regional variiert und eine Größe von 32mm bis 43mm im erigierten Zustand im Durchschnitt annimmt. Dies sind nur Penisdurchmesser- Durchschnittswerte, demnach können die Penisdurchmesser durchaus auch Größen von 60mm oder 10mm aufweisen.

In einer weiteren Ausführungsform ist die Penismanschette dadurch gekennzeichnet, dass sich der Hohlkörper bereichsweise entlang seiner Längsachse im Innendurchmesser verjüngt und der Innendurchmesser an einem Hohlkörperende kleiner ist als an einem zweiten Hohlkörperende. Dies erweist sich als besonders vorteilhaft für die Positionierung der Penismanschette an einen Penis, dessen Durchmesser am Penisschaft im Bereich des Beckens größer ist als an der Peniseichel.

In einer weiteren Ausführungsform der Penismanschette verjüngt sich der Hohlkörper bereichsweise entlang seiner Längsachse im Innendurchmesser. Der Innendurchmesser ist somit an einem Hohlkörperbereich kleiner ist als an einem zweiten Hohlkörperbereich. Dies erweist sich vorteilhaft für Positionierung der Penismanschette an einen Penis der bereichsweise im Durchmesser kleiner und/oder größer ausgestaltet ist.

In einer weiteren bevorzugten Ausführungsform der Erfindung ist die Penismanschette dadurch gekennzeichnet, dass der Hohlkörper eine Wanddicke von 1 mm bis 60 mm, bevorzugt von 15mm bis 30 mm aufweist. Es war überraschend, dass die bevorzugten Materialstärken ein besonders natürliches Gefühl beim Träger einer Penismanschette und beim Sexualpartner hervorruft. Zudem erweist sich die Lebensdauer der Penismanschette dadurch vorteilhaft langlebig.

In einer weiteren bevorzugten Ausführungsform ist die Penismanschette dadurch gekennzeichnet, dass die Klammer eine Wanddicke von 0,5 mm bis 15 mm, bevorzugt von 1 mm bis 5 mm aufweist. Es hat sich überraschend gezeigt, dass die Klammer dadurch der Penismanschette genau bei diesen Werten ein optimal elastisches Verhalten zuführt. Aufgrund dessen wird eine sehr gute Klammerkraft für die Positionierung an den Penisschaft bereitgestellt.

In einer weiteren bevorzugten Ausführungsform ist die Penismanschette dadurch gekennzeichnet, dass die Wanddicke des Hohlkörpers nicht konstant ist. Die Wanddicke kann beispielsweise über die Längsachse des Hohlkörpers inhomogen bzw. nicht konstant sein. Eine nicht konstant ausgebildete Wanddicke über die gesamte Länge ermöglicht besonders natürliche Formen der Außenfläche.

In einer weiteren bevorzugten Ausführungsform verändert sich die Wanddicke radial im Querschnitt, sodass besonders bevorzugt die Wanddicke den größten Wert auf der gegenüberliegenden Seite des Schlitzes aufweist. Eine derartige sichelförmige Ausgestaltung erweist sich in gleicher Weise vorteilhaft, wie die Ausgestaltung der schon beschriebenen Schichtdicken. Die sichelförmige Wanddicke erweist sich enorm stabiltätssteigernd und ermöglicht eine erhöhte Lebensdauer der Manschette. Zudem wirkt die vergrößerte Materialanhäufung auf der gegenüberliegenden Seite des Schlitzes stimulierend für einen Sexualpartner beim Geschlechtsverkehr, da die Manschette vorzugsweise radial über den größtmöglichen Umfang der Peniseichel hinausragt. Neben der Stimulation des Sexualpartners hat sich vorteilhaft gezeigt, dass ein vergrößerter einseitiger Druck über die Seite mit der größten Wanddicke auf den Penis, während des Ein- und Ausführens des Penis in eine Körperöffnung, übertragen wird, welcher sich vorteilhaft stimulierenden für den Manschettenträger auswirken kann.

In einer weiteren Ausführungsform ist die Penismanschette dadurch gekennzeichnet, dass die Wanddicke im Querschnitt des Hohlkörpers ausgehend von der vom Schlitz gegenüberliegenden Seite zum Schlitz hin radial abnimmt.

In einer weiteren bevorzugten Ausführungsform weist die Wanddicke auf der gegenüberliegenden Seite des Schlitzes einen Wert von bevorzugt mindestens 10 mm, stärker bevorzugt mindestens 15 mm, besonders stark bevorzugt mindestens 18mm, vielmehr bevorzugt mindestens 20mm und insbesondere mindestens 30mm auf. Durch vorbenannte Werte wird der Penisdicke soweit künstlich vergrößert, dass ein Sexualpartner besonders stark stimuliert werden kann. So kann die Manschette auch bei besonders dünnen Penissen zu einem gewünschten Erfolg führen.

In einer weiteren bevorzugten Ausführungsform weist die Wanddicke auf der gegenüberliegenden Seite des Schlitzes einen Wert von bevorzugt maximal 80 mm, stärker bevorzugt maximal 70 mm, besonders stark bevorzugt maximal 50mm, vielmehr bevorzugt maximal 40mm und insbesondere maximal 35mm.

In einer weiteren bevorzugten Ausführungsform verändert sich die Wanddicke des Hohlkörpers entlang der seiner Längsachse. In beiden Endbereichen des Hohlkörpers sind bevorzugt kleine Wanddicken vorgesehen, wobei diese sich beide nach außen entlang der Längsachse verjüngen. Die gegenüberliegende Seite des Schlitzes weist dabei weiterhin die größtmögliche Dicke der Manschette auf.

Es ist dabei nicht ausgeschlossen, dass die Wanddicke des Hohlkörpers bereichsweise auch konstant verläuft.

In einer bevorzugten Ausführungsform ist die Penismanschette so ausgestaltet, dass die Wanddicke auf der vom Schlitz gegenüberliegenden Seite am größten ist, wobei sich die Dicke zum Schlitz hin verkleinert. Dies verhindert vorteilhaft störende Kanten, insbesondere wenn die Wanddicke nicht abrupt, sondern radial linear oder exponentiell abnimmt.

In einer weiteren bevorzugten Ausführungsform ist die Wanddicke der Klammer bevorzugt konstant ausgestaltet. Dies wirkt sich vorteilhaft auf die Klammerkraft aus, da diese unter anderem von der Wanddicke abhängt und somit über dies gesamte Klammer gleich ist.

In einer weiteren bevorzugten Ausführungsform ist die Penismanschette dadurch gekennzeichnet, dass die Wanddicke des Hohlkörpers im Bereich des Schlitzes und an den Hohlkörperenden abnimmt. Dadurch weist die Penismanschette vorteilhaft keine störenden Kanten auf.

In einer weiteren bevorzugten Ausführungsform ist die Penismanschette dadurch gekennzeichnet, dass die Außenfläche des Hohlkörpers eine glatte und/oder eine für den

Geschlechtsverkehr Stimulanz fördernde Oberfläche aufweist, beispielsweise durch Noppen, Äderungen, örtliche Verdickungen und/oder Rillen. Eine besonders glatte Außenfläche wirkt vorteilhaft zum Einführen des Penis ausgestattet mit der Penismanschette in eine Körperöffnung, da hier kein Widerstand durch eine ausgeprägte Oberfläche vorliegt. Noppen, Äderungen, örtliche Verdickungen oder Rillen wirken hingegen besonders vorteilhaft stimulierend für den Sexualpartner.

In einer weiteren Ausführungsform ist die Außenfläche mit zusätzlichen Reizarmen ausgestattet. Derartige Reizarme können beispielsweise vorteilhaft die Klitoris einer Frau zusätzlich stimulieren.

In einer weiteren bevorzugten Ausführungsform ist die Penismanschette dadurch gekennzeichnet, dass die Penismanschette farbig ausgestaltet ist, bevorzugt in rosa, braun, schwarz, blau, rot, hautfarben und/oder grün.

In einer weiteren bevorzugten Ausführungsform ist die Farbe der Penismanschette ausgewählt aus der Gruppe umfassend schwarz, schwarz metallic, weiß, grün, grün metallic, oliv, oliv metallic, gold, silber, grau, hautfarbe, gelb, neon gelb, orange, neon orange, rot, bordeaux, pflaume, aubergine, petrol, violett, blau, blau metallic, pink, neon pink, braun, transparent, rosa, flieder, lila, magenta, türkis, amethyst, bronze und/oder bronze metallic.

Es hat sich besonders vorteilhaft gezeigt, dass sog. Hygieneflecken wie beispielsweise Sperma oder Vaginalflüssigkeit auf den genannten Farben als weniger abstoßend bzw. anstößig wahrgenommen werden. Selbst Paare oder Gruppen, die sich bereits über einen längeren Zeitraum kennen und sexuelle Interaktionen umsetzen, empfinden die genannten Hygieneflecken als einschränkend und nachteilig. Im ungünstigsten Falle kann dies zu Stressreaktionen führen. Die Ausschüttung von Stresshormonen beeinflusst aber die sexuellen Aktivitäten bei Männern und Frauen gleichermaßen nachteilig. Ein entsprechend hoher Level an Stresshormonen im Blut verhindert sogar eine erfolgreiche Befruchtung.

In einer weiteren bevorzugten Ausführungsform verfügt die Penismanschette über eine Vibrationsfunktion. Die Vibrationsfunktion wird bevorzugt durch einen kleinen batteriebetriebenen Motor ermöglicht. Dieser ist in einer besonders bevorzugten Alternative mit dem Stützskelett in Verbindung und regt dieses zu einer Schwingung bzw. Vibration an. Dadurch, dass das Stützskelett über den gesamten Hohlkörper gleichmäßig verteilt vorliegt, entsteht vorteilhaft eine Vibration in der gesamten Penismanschette. Es hat sich gezeigt, dass eine solche Vibrationsfunktion zu einer erhöhten Stimulanz sowohl des Trägers der Penismanschette als auch des Sexualpartners führt.

In einem weiteren Aspekt der Erfindung wird die Penismanschette der eingangs genannten Art als Manschette für einen Penis beim Geschlechtsverkehr verwendet. Die Penismanschette wirkt sich durch die schon oben beschriebenen Vorteile stimulierend auf den Träger einer Penismanschette und des Sexualpartners. Insgesamt wirkt ein verbessertes Sexualleben stressabbauend und führt unter Umständen auch zu einer verbesserten Empfängnisbereitschaft der Frauen, sodass selbst beim Vorliegen von medizinischen zum Teil pathologischen Problemen ein Kinderwunsch realisiert werden kann, wenn die Partner vielfältige sexuelle Praktiken umsetzen.

Der Fachmann erkennt, dass bevorzugte Ausführungsformen und Vorteile, welche im Zusammenhang mit der Penismanschette offenbart wurden sich gleichermaßen auf die beanspruchte Verwendung übertragen. Insbesondere gelten die bevorzugten Ausführungsformen für die Penismanschette gleichermaßen für deren Verwendung.

In einer weiteren Ausführungsform ist die Verwendung der Penismanschette dadurch gekennzeichnet, dass
a) der Schlitz der Penismanschette durch Krafteinwirkung vergrößert wird;
b) ein Penisschaft durch den Schlitz geführt wird;
c) die Penismanschette an den Penisschaft angelegt wird, wobei die Innenfläche des Hohlkörpers am Penisschaft aufliegt und der Schlitz kontinuierlich durch die Krafteinwirkung vergrößert bleibt;
d) die Krafteinwirkung aufgehoben wird, wobei eine rückwirkende Klammerkraft die Penismanschette um den Penisschaft positionsfest hält.

In einer weiteren bevorzugten Ausführungsform wird die Penismanschette der eingangs genannten Art bevorzugt an der linken oder rechten, stärker bevorzugt an der unteren oder oberen Penisschaft-Seite angebracht.

In einer weiteren Ausführungsform ist die Verwendung dadurch gekennzeichnet, dass ein Kondom über einen mit der Penismanschette, der eingangs genannten Art umklammerten Penis gestülpt wird und die Penismanschette zwischen Penisschaft und dem Kondom angeordnet ist.

Die erfindungsgemäße Penismanschette soll im Folgenden anhand von Beispielen näher erläutert werden, ohne auf diese Beispiele beschränkt zu sein.

### FIGUREN

### Kurzbeschreibung der Abbildungen

- **Fig.** 1: Darstellung einer bevorzugten Penismanschette
- **Fig. 2**: Darstellung eines Penis, welcher eine um den Penisschafft angelegte bevorzugte Penismanschette aufweist
- **Fig. 3**: Darstellung eines Penis von einer Unterseite, wobei der Penisschaft durch eine bevorzugte Penismanschette umklammert ist
- **Fig. 4**: Schnittdarstellung eines Penis mit angelegter bevorzugter Penismanschette
- **Fig. 5**: Darstellung einer bevorzugten Klammer von einer Vorderseite
- **Fig. 6**: Darstellung einer bevorzugten Klammer von einer Rückseite

### Detaillierte Beschreibung der Abbildungen

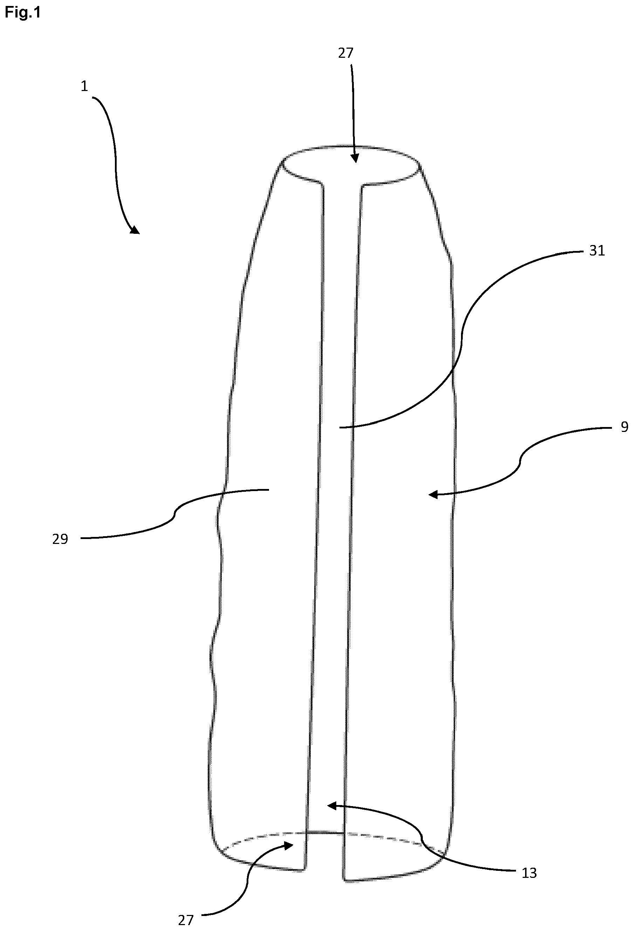

Fig. 1 illustriert eine bevorzugte Penismanschette **1** in einer Draufsicht. Die Penismanschette **1** weist einen hülsenförmigen Hohlkörper **9** mit jeweils einer stirnseitigen Öffnung **17** an beiden Hohlkörperenden auf, wobei die Länge des Hohlkörpers **9** größer ist als sein Innendurchmesser **9.** Die Penismanschette **1** ist im Nichtgebrauch und dementsprechend im Ausgangszustand ohne Einwirkung von Elastizität dargestellt. Die Spaltbreite des Schlitzes **13** weist in diesem Zustand bevorzugt eine Größe von bevorzugt 25mm bis 40mm auf. Der Hohlkörper **9** umfasst ferner Silikon. Die Außenfläche **29** ist dabei in ihrer Formgestaltung frei ausgestaltbar. So kann die Außenfläche **29** des Hohlkörpers **9** beispielsweise Noppen, Äderungen, örtliche Verdickungen und/oder Rillen aufweisen. Überdies ist die Penismanschette bevorzugt farbig ausgestaltet: Zum Beispiel in rosa, braun, schwarz, blau, rot, hautfarben und/oder grün.

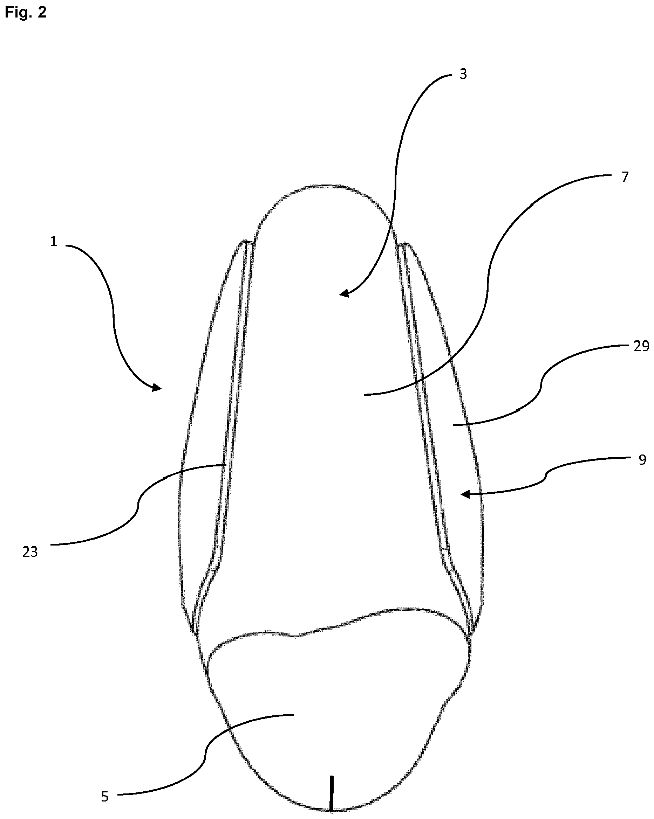

Fig. 2 stellt einen Penis **3** dar, der an seinem Penisschaft **7** von einer bevorzugten Ausführungsform der Penismanschette 1 umklammert ist. Die Penismanschette **1** ist demnach in seiner Verwendung bzw. in seinem Gebrauchszustand illustriert. Für das Umklammern des Penis **3** wird zunächst Schlitz **13** der Penismanschette **1** durch Krafteinwirkung vergrößert. In einem nächsten Schritt wird der Penisschaft **7** durch den Schlitz **13** seitlich durchgeführt. Anschließend wird die Penismanschette **1** an den Penisschaft **7** angelegt, wobei die Innenfläche **31** des Hohlkörpers **9** am Penisschaft **7** aufliegt und der Schlitz **13** kontinuierlich durch die Krafteinwirkung vergrößert bleibt. In einem letzten Schritt wird die Krafteinwirkung aufgehoben, wobei eine rückwirkende Klammerkraft die Penismanschette **1** um den Penisschaft **7** positionsfest hält. Die Kraft wird dabei bevorzugt durch menschliche Hände aufgebracht. Die Längsausdehnung des Hohlkörpers **9** gleicht im Wesentlichen der Länge eines Penisschafts **7** und weist vorzugsweise eine Längsausdehnung von 30mm - 250mm. In der bevorzugten Ausführungsform bleibt die Peniseichel **5** sowie eine große Fläche des Penisschafts **7** frei von Fremdmaterial, sodass dem Träger der bevorzugten Manschette ein besonders natürliches Gefühl beim Geschlechtsverkehr erfährt. Die freie Penisfläche weist einen Anteil von 10% - 35 % in Bezug auf die äußere Gesamtfläche des Penis, welche ohne Penismanschette sichtbar wäre. Der Hohlkörper **9** weist bevorzugt eine Wanddicke **23** bevorzugt von 15 bis 30 mm auf. An örtlichen Stellen kann die Wanddicke auch bis zu 60mm betragen. So besteht die Möglichkeit die Penismanschette mit verschiedenen Reizarmen an der Außenfläche **29** auszugestalten, die bspw. stimulierend auf die Klitoris einer Frau während des Geschlechtsverkehrs wirken sollen.

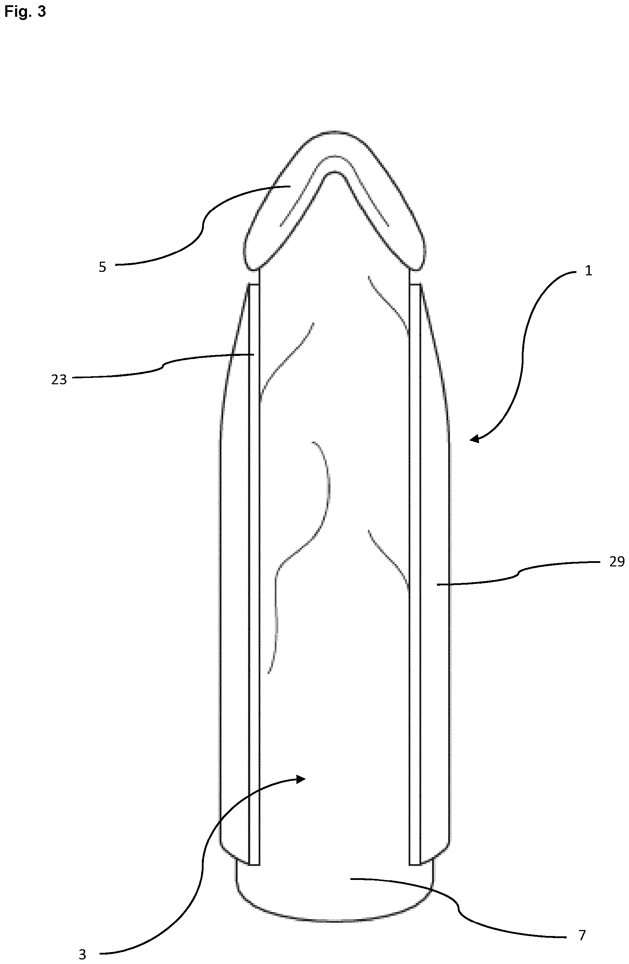

Fig. 3 ist eine Darstellung eines Penis **3** von einer Unterseite, wobei der Penisschaft **7** durch eine bevorzugte Penismanschette **1** umklammert ist. Im Vergleich zu Fig. 2 befindet sich die Penismanschette **1** auf der gegenüberliegenden Seite des Penis **3.** Die Penismanschette **1** ist dabei variabel an jeder Seite eines Penisschafts **7** anbringbar.

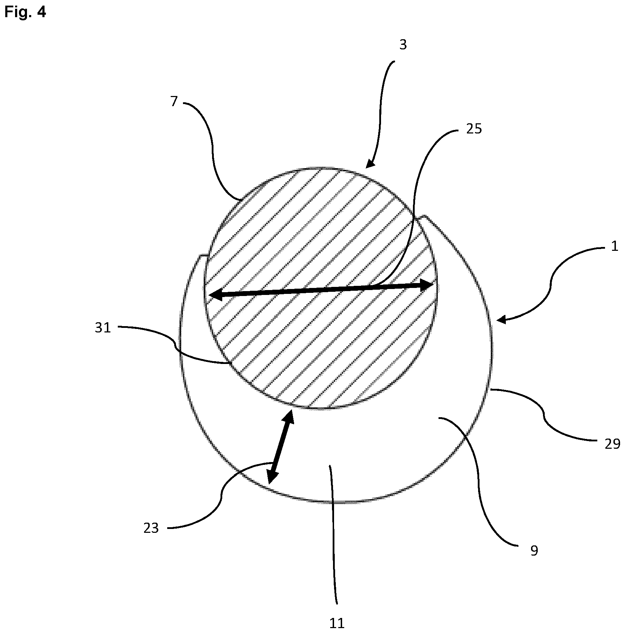

Fig. 4 ist eine schematische Schnittdarstellung eines Penis 3 mit angelegter bevorzugter Penismanschette **1.** Der Innendurchmesser **25** des Hohlkörpers **9** entspricht dabei in angelegter Position dem Durchmesser des Penis **3.** Statistiken haben ergeben, dass der durchschnittliche Penisdurchmesser regional variiert und eine durchschnittliche Größe von 32mm bis 43mm im erigierten Zustand annimmt. Beim Nichtgebrauch der Penismanschette **1** weist der Innendurchmesser **25** bevorzugt eine Größe von 25 mm bis 33mm auf. In der Schnittdarstellung ist deutlich das C-Profil **11** der Penismanschette **1** zu sehen. Die dargestellte Ausführungsform weist hingegen in dem dargestellten Bereich kein Stützskelett auf. Die Wanddicke **23** des Hohlkörpers **9** ist radial betrachtet nicht konstant. Die Wanddicke **23** variiert bevorzugt von 1mm bis 60 mm, wobei die größte Wanddicke **23** auf der gegenüberliegenden Seite des Schlitzes **13** vorliegt.

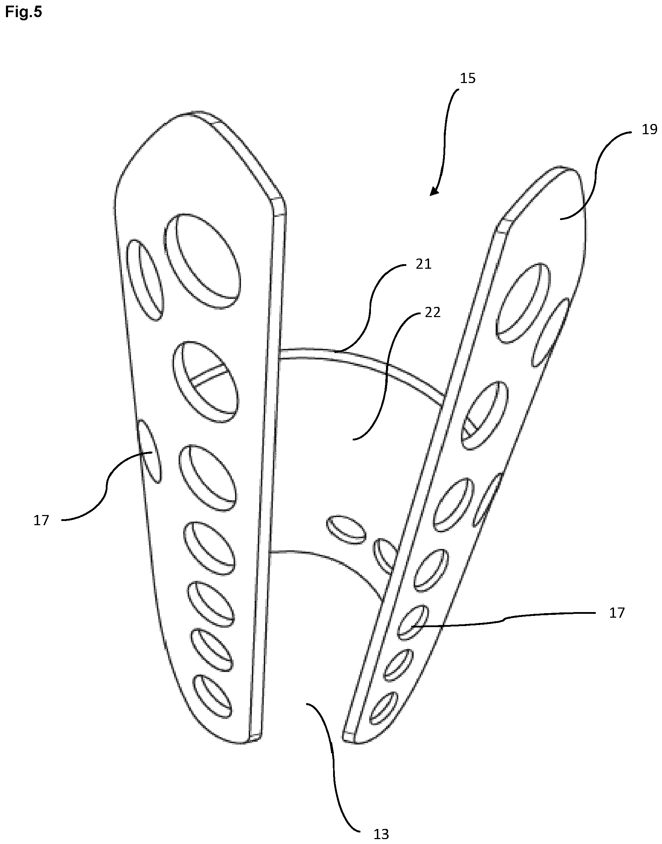

Fig. 5 ist die Darstellung einer bevorzugten Klammer **15** von einer Vorderseite. Die Klammer **15** weist in einem unteren Bereich einen Querschnitt, ausgestaltet in einem C-Profil **22.** Der untere Bereich, welcher das C-Profil **22** aufweist, ist in seiner Längsausdehnung bevorzugt so groß ausgestaltet wie 1/3 bis 1/2 der gesamten Längsausdehnung der Klammer. Ausgehend des C-Profils **22** umfasst die Klammer **15** bevorzugt zwei Fortsätze **19,** die in axialer Richtung ausgebildet sind. Die Wanddicke **21** der Klammer **15** ist konstant. Ferner weist die Klammer **15** eine Vielzahl von Öffnungen **17** auf, die gleichmäßig über die gesamte Klammer **15** verteilt sind. Bei Einwirkung zweier Kräfte (Druck oder Zug) entgegengesetzter Richtung auf die Schenkel des C-Profil **22** entsteht eine Klammerkraft (Federkraft), da das Material bzw. die Klammer **15** durch sein elastisches Verhalten zurück in die Ausgangsposition gelangen will. Die Klammer **15** wirkt für die Penismanschette **1** als ein Stützskelett und definiert das stabile und elastische Verhalten des Penismanschette **1.** Dabei ist die Klammer **15** bevorzugt in Silikon eingegossen/ eingebettet, sodass die Klammer **15** vollständig mit einer Schichtdicke von 1 mm bis 50mm, stärker 2 mm bis 25mm Silikon ummantelt ist.

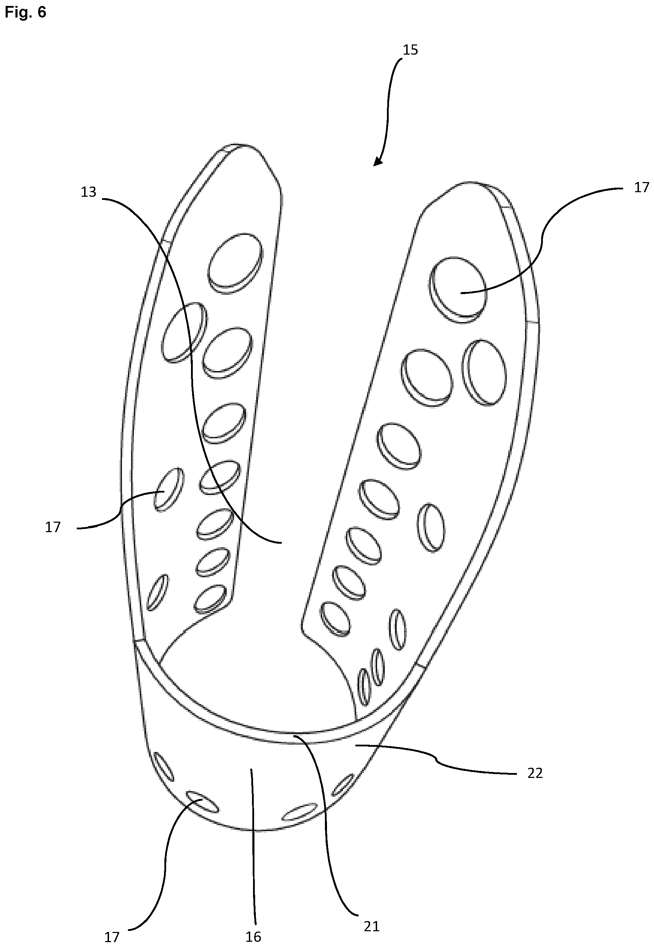

Fig. 6 ist die Darstellung einer bevorzugten Klammer **15** aus Fig. 5 von einer Rückseite.

### Bezugszeichenliste

- 1: Penismanschette
- 3: Penis
- 5: Peniseichel
- 7: Penisschaft
- 9: Hohlkörper
- 11: C-Profil des Hohlkörpers
- 13: Schlitz bzw. Klammerschlitz
- 15: Klammer
- 16: Nach außen gewandte Seitenfläche der Klammer
- 17: Klammeröffnungen
- 19: Klammerfortsätze
- 21: Wanddicke der Klammer
- 22: C-Profil der Klammer
- 23: Wanddicke des Hohlkörpers
- 25: Innendurchmesser des Hohlkörpers
- 27: Stirnseitige Öffnung des Hohlkörpers
- 29: Außenfläche des Hohlkörpers
- 31: Innenfläche des Hohlkörpers

## Patentansprüche

1. Penismanschette (1), aufweisend einen hülsenförmigen Hohlkörper (9) mit jeweils einer stirnseitigen Öffnung (17) an beiden Hohlkörperenden, wobei die Länge des Hohlkörpers (9) größer ist als sein Innendurchmesser (25) und der Hohlkörper (9) im Querschnitt mindestens bereichsweise als ein C-Profil (11) ausgestaltet ist, wodurch der Hohlkörper (9) entlang seiner Längsachse einen Schlitz (13) aufweist
**dadurch gekennzeichnet, dass**
der Hohlkörper (9) auf der gegenüberliegenden Seite des Schlitzes (13) eine Wanddicke von mindestens 8 mm aufweist,

2. Penismanschette (1) nach Anspruch 1
**dadurch gekennzeichnet, dass**
die Wanddicke im Querschnitt des Hohlkörpers ausgehend von der vom Schlitz gegenüberliegenden Seite zum Schlitz hin radial abnimmt.

3. Penismanschette (1)) nach einem der vorhergehenden Ansprüche
**dadurch gekennzeichnet, dass**
der Hohlkörper (9) Silikon umfasst.

4. Penismanschette (1) nach einem der vorhergehenden Ansprüche
**dadurch gekennzeichnet, dass**
der Hohlkörper (9) ein eingebettetes Stützskelett umfasst.

5. Penismanschette (1) nach einem der vorhergehenden Ansprüche
**dadurch gekennzeichnet, dass**
das Stützskelett eine aus einem einzigen Bauteil ausgestaltete Klammer (15) ist, wobei die Klammer (15) bereichsweise ebenfalls einen als C-Profil (22) ausgestalteten Querschnitt aufweist wodurch die Klammer (15) entlang ihrer Längsachse auch einen Klammerschlitz (13) aufweist.

6. Penismanschette (1) nach einem der vorhergehenden Ansprüche
**dadurch gekennzeichnet, dass**
die Klammer (15) vollständig mit einer Schichtdicke von 1mm bis 50mm, stärker 2mm bis 25mm Silikon ummantelt ist, wobei die Klammer (15) auf ihrer nach außen gewandten Seitenfläche (16) der gegenüberliegenden Seite des Schlitzes (13) eine Schichtdicke von mindestens 5mm aufweist auf.

7. Penismanschette (1) nach einem der vorhergehenden Ansprüche
**dadurch gekennzeichnet, dass**
die Klammer (15) eine Vielzahl von Öffnungen (17) aufweist, wobei die Öffnungen (17) bevorzugt gleichmäßig über die gesamte Klammer (15) verteilt sind.

8. Penismanschette (1) nach einem der vorhergehenden Ansprüche
**dadurch gekennzeichnet, dass**
die Klammer (16) aus einem Material gefertigt ist, welches ein Stützmaterial, bevorzugt Holz, Metall oder Kunststoff, umfasst.

9. Penismanschette (1) nach einem der vorhergehenden Ansprüche
**dadurch gekennzeichnet, dass**
die Längsausdehnung des Hohlkörpers (9) im Wesentlichen der Länge eines Penisschafts (7) gleicht, bevorzugt eine Längsausdehnung von 30mm - 250mm.

10. Penismanschette (1) nach einem der vorhergehenden Ansprüche
**dadurch gekennzeichnet, dass**
der Innendurchmesser (25) des Hohlkörpers (9) eine Größe von 20 mm bis 40 mm, bevorzugt 25 mm bis 33mm aufweist.

11. Penismanschette (1) nach einem der vorhergehenden Ansprüche
**dadurch gekennzeichnet, dass**
der Hohlkörper (9) bereichsweise eine Wanddicke (23) von 1 mm bis 60 mm, bevorzugt von 15 bis 30 mm aufweist.

12. Penismanschette (1) nach einem der vorhergehenden Ansprüche
**dadurch gekennzeichnet, dass**
die Wanddicke (23) des Hohlkörpers (9) nicht konstant ist und

13. Penismanschette (1) nach einem der vorhergehenden Ansprüche
**dadurch gekennzeichnet, dass**
eine Wanddicke (23) des Hohlkörpers (9) an den Hohlkörperenden abnimmt.

14. Penismanschette (1) nach einem der vorhergehenden Ansprüche
**dadurch gekennzeichnet, dass**
eine Außenfläche (29) des Hohlkörpers (9) eine glatte und/oder eine für den Geschlechtsverkehr Stimulanz fördernde Oberfläche aufweist, beispielsweise durch Noppen, Äderungen, örtliche Verdickungen und/oder Rillen.

15. Verwendung der Penismanschette (1) gemäß einem der vorherigen Ansprüche als Manschette für einen Penis (3) beim Geschlechtsverkehr.
